(19) Europäisches Patentamt

European Patent Office

Office européen des brevets

(11) **EP 1 666 024 A1**

(12) **EUROPEAN PATENT APPLICATION**
published in accordance with Art. 158(3) EPC

(43) Date of publication:
**07.06.2006 Bulletin 2006/23**

(51) Int Cl.:
***A61K 9/02*** (1968.09)    ***A61K 9/06*** (1968.09)
***A61K 47/38*** (1990.01)

(21) Application number: **04772819.1**

(22) Date of filing: **30.08.2004**

(86) International application number:
**PCT/JP2004/012870**

(87) International publication number:
**WO 2005/020960 (10.03.2005 Gazette 2005/10)**

(84) Designated Contracting States:
**AT BE BG CH CY CZ DE DK EE ES FI FR GB GR HU IE IT LI LU MC NL PL PT RO SE SI SK TR**

(30) Priority: **29.08.2003 JP 2003306599**

(71) Applicant: **Sato Pharmaceutical Co. Ltd.**
**Tokyo 107-0051 (JP)**

(72) Inventors:
• **NISHIJIMA, Satoshi,**
**R&D Ctr.,**
**Sato Pharma Co, Ltd**
**Tokyo 140-0011 (JP)**
• **KATAYAMA, Masahide,**
**R&D Ctr,**
**Sato Pharma Co., Ltd**
**Tokyo 140-0011 (JP)**

• **TATARA, Mitsutoshi,**
**R&D Ctr.,**
**Sato Pharma Co, Ltd**
**Tokyo 140-0011 (JP)**
• **SHIMIZU, Toshihito,**
**R&D Ctr.,**
**Sato Pharma Co, Ltd**
**Tokyo 140-0011 (JP)**

(74) Representative: **Ziebig, Marlene et al**
**Anwaltskanzlei**
**Gulde Hengelhaupt Ziebig & Schneider**
**Wallstrasse 58/59**
**10179 Berlin (DE)**

(54) **PREPARATION FOR RECTAL ADMINISTRATION**

(57)    A pharmaceutical preparation for rectal administration by which a drug is retained in the affected region or the lower region of the rectum, where the drug is sustained-released is disclosed. The present invention is a pharmaceutical preparation for rectal administration comprising a coated drug-supported particle dispersed in a base, wherein the coated drug-supported particle is what a drug-supported porous microparticulate carrier is coated with a water-soluble polymer having a certain viscosity.

EP 1 666 024 A1

## Description

TECHNICAL FIELD

The present invention relates to a pharmaceutical preparation for rectal administration, more particularly to a suppository and an injection type ointment by which a drug is retained in the affected region of the rectum and the release of the drug is controlled.

BACKGROUND ART

A suppository is, generally, what a drug is mixed with or dissolved in a base to form into a certain shape. When the suppository is applied to an anal, the base is melted by the body temperature or it is dissolved with the secretion, the drug is released, and then absorbed through the mucosa. In this case, if the drug is gradually released from the suppository for a long time, the side effect due to over-release of the drug and the troublesomeness because of frequent administration may be decreased. Several sustained-release suppositories, therefore, have been developed. See, for example, JP-A-5-238930 and JP No. 2702938 (the term "JP-A" as used herein means an "unexamined published Japanese patent application").

In JP-A-5-238930, the use of a drug sustained-release carrier in a suppository is disclosed, wherein the carrier is formed by allowing a drug to be adsorbed on a porous microparticulate carrier, and then causing a mixture of a higher fatty acid glyceride and a water-soluble high molecular substance such as a polyethylene glycol, etc. to be adsorbed on it.

Moreover, in JP No. 2702938, it is disclosed that a sustained-release suppository can be prepared by mixing a drug and a water-soluble high molecular substance such as a hydroxyalkyl cellulose, etc. to form a solid preparation such as a granule, etc., coating the surface of the solid preparation, and then dispersing it into a base.

For the purpose of local curing (local action), such as curing of hemorrhoids or the like, however, when the suppository described in JP-A-5-238930 is administered to the rectum, the suppository is melted by the body temperature or the secretion and the drug sustained-release carrier is eluted into the rectum, but the viscosity of the polyethylene glycol adsorbed on the surface of the drug sustained-release carrier is so low that the carrier cannot be sufficiently adhered to and retained on the mucosa of the affected region in the rectum, and then the drug sustained-release carrier is moved to the upper region of the rectum over the passage of time. There is the problem, therefore, that said event results in a decrease in the amount of the drug sustained-release carrier present at the affected region of the rectum, the amount of the drug which should be released from the drug sustained-release carrier to the affected region of the rectum is decreased, and then the efficacy of the drug cannot be sufficiently exerted. Further, in the case of a suppository for the purpose of systemic action, if a drug is absorbed from the lower region of the rectum, the drug doesn't pass through the liver so that the inactivation of the drug due to the metabolism can be decreased. However, if a drug is absorbed from the upper region of the rectum, there is the problem that the drug passes through the liver so that the first pass effect occurs to result in a decrease in the amount of the drug to be appeared in the systemic circulation. Specifically speaking, in the examples of JP-A-5-238930, polyethylene glycol 6000 is adsorbed on the surface of the drug sustained-release carrier, but this is only for the purpose of the drug release control. Since the viscosity of polyethylene glycol 6000 is so low (see reference examples described below), the drug sustained-release carrier coated with polyethylene glycol 6000 cannot be sufficiently adhered to or retained in the affected region of the rectum or the mucosa of the lower region of the rectum.

Moreover, JP No. 2702938 discloses that a suppository which can be prepared by coating a granular solid preparation to be dispersed on it. However, JP No. 2702938 does not disclose the coating agents. Furthermore, in the examples, the production of the suppository dispersed with the coated solid preparation is not carried out. In addition, the coating on the surface of the solid preparation is the one to control the releasing rate of the drug and it is not intended to allow the solid preparation to be retained in the affected region of the rectum. Therefore, when the suppository wherein the coated granular solid preparation described in JP No. 2702938 is dispersed therein is administered to the rectum for the purpose of local action at the rectum, as in the case of the suppository described in JP-A-5-238930, the solid preparation is eluted from the suppository into the rectum, but the solid preparation is moved to the upper region of the rectum. Therefore, there is the problem that said event results in a decrease in the amount of the solid preparation present at the affected region of the rectum, the amount of the drug which should be released from the solid preparation to the affected region of the rectum is decreased, and then the efficacy of the drug cannot be sufficiently exerted. Moreover, in the case for the purpose of systemic action, as in the case of the suppository described in JP-A-5-238930, there is the problem that the solid preparation is moved to the upper region of the rectum, where the drug is absorbed, and then the first pass effect due to the passage through the liver occurs.

Further speaking, the solid preparation dispersed in the suppository of JP No. 2702938 includes, for example, a granule, as mentioned above, which has a particle size of from about 500 to about 1400 μm. In such a large particle size, there is the problem that the solid preparation which has released by the melting of the suppository is dispersed such that large gaps are formed throughout the affected region of the rectum, the affected region at which any solid preparation may not be present occurs widely, and then the drug can not be uniformly sustained-released throughout the affected region. Moreover, since the particle size of the solid preparation is large, there is the problem that the surface area of one particle of the solid preparation is smaller as compared with that of the microparticle preparation which has greater number of particles whose particle size is decreased such that the amount of drugs may be same as the amount of drugs in one particle of the solid preparation, the amount of drugs which should be released throughout the affected region is decreased, and then the efficacy of drugs cannot be exerted. In addition, since the particle size is large, when the solid preparation is mixed with the base to form the suppository, the solid preparation is sedimented while the suppository solidifies to result in the suppository in which the solid preparation is ununiformly dispersed. Thus, when such suppository is melted by the body temperature or the secretion, there is the problem that a constant amount of drugs cannot be supplied to the affected region of the rectum, where greater amount of the solid preparation may be eluted or smaller amount of it may be eluted. Further speaking, since the solid preparation is ununiformly dispersed, there is the problem that the strength of the suppository is declined to lead to the occurrence of cracks or the like of the suppository. Moreover, since the particle size of the solid preparation is large, there is the problem that the coating of the solid preparation is damaged during producing, thereby the effect of the coating of sustained-releasing drugs contained in the solid preparation cannot be obtained.

DISCLOSURE OF THE INVENTION

The present invention is made in view of such problems which prior arts have, thus it is an object of the present invention to provide a pharmaceutical preparation for rectal administration by which a drug-supported particle is adhered to and retained in the affected region, where the drug is sustained-released, thereby the efficacy of the drug is sufficiently exerted at the affected region, and which can suppress side effects and allows the administration frequency of the preparation to decrease. Moreover, another object of the present invention is to provide a pharmaceutical preparation for rectal administration by which a drug-supported particle is adhered to and retained in the lower region of the rectum, where the drug is sustained-released, thereby liver first pass effect is not affected.

Further, another object of the present invention is to provide a pharmaceutical preparation for rectal administration by which a drug-supported particle is uniformly adhered and retained throughout the affected region of the rectum, where the drug is released evenly over the affected region while the drug is sustained-released. Moreover, additional another object of the present invention is to provide a suppository and the like wherein a coating layer of a drug-supported particle is not damaged during the production of the suppository and the drug-supported particle is uniformly dispersed in a base to provide a suppository which is hardly cracked.

The present inventors have made various studies to solve the above problems and have consequently found that the above problems can be solved by allowing a drug to be supported by a porous microparticulate carrier, and coating the drug-supported particle with a water-soluble polymer having a certain viscosity, and by making the particle size of the coated drug-supported particle into a certain size, and then they have accomplished the present invention.

That is to say, the present invention is a pharmaceutical preparation for rectal administration comprising a coated drug-supported particle dispersed in a base, characterized in that said coated drug-supported particle is what a drug-supported porous microparticulate carrier is coated with a water-soluble polymer having a viscosity of from 0.1 to 10.0 Pa.s (pascal second) under the condition using 20 % by weight aqueous solution of the water-soluble polymer at 37 °C.

Moreover, in the present invention, the drug-supported particle preferably has a particle size of from 0.1 to 300 μm.

BRIEF DESCRIPTION OF THE DRAWINGS

Figure 1 is a photograph showing the ability of adhesion and retention to cellulose tube of the suppositories of the example 1 and the comparative example 1.
Figure 2 is a graph showing the time-dependent change of the concentration of lidocaine hydrochloride in rat rectal tissue after the administration of the suppository of the example 1 or the comparative example 1 to rat.

BEST MODE FOR CARRYING OUT THE INVENTION

The pharmaceutical preparation for rectal administration of the present invention is explained below.
The porous microparticulate carrier used in the present invention is a microparticle having a large number of fine pores at least on its surface. The structure of the porous microparticulate carrier can be particle-aggregated type, bubble-dispersed type, network structure type or the like, it preferably has a particle size of from 0.1 to 200 $\mu$m, a specific surface area of from 100 to 1000 $m^2$/g, and an absorbability of oil of from 0.5 to 8.0 mL/g. The main component of the porous microparticulate carrier is preferably silicic acids, but is not particularly limited as long as it can support drugs. The porous microparticulate carrier used in the present invention has more preferably a particle size of from 0.1 to 100 $\mu$m, a specific surface area of from 200 to 700 $m^2$/g, and an absorbability of oil of from 2.0 to 7.0 mL/g. Specifically, the porous microparticulate carrier includes light silicic acid anhydride, magnesium aluminometasilicate, magnesium silicate, and hydrous silicon dioxide. Light silicic acid anhydride and magnesium aluminometasilicate are more preferable. These porous microparticulate carriers are commercially available and therefore are easily available. Further, the porous microparticulate carrier can be used alone, or in combination of two or more carriers.

The drug supported by porous microparticulate carrier includes, but not limited to adrenocortical hormone agent, local anesthetic agent, anti-inflammatory agent, antipruritic agent, wound healing agent, vitamin agent, sulfa drug, germicide, antihistamic agent and the like. The specific examples of adrenocortical hormone agent include prednisolone acetate, prednisolone, hydrocortisone acetate, hydrocortisone, cortisone acetate, cortisone, dexamethasone acetate, dexamethasone, hydrocortisone butyrate, prednisolone acetate valerate or triamcinolone acetate. The specific examples of local anesthetic agent include lidocaine hydrochloride, lidocaine, dibucaine hydrochloride, dibucaine, procaine hydrochloride, procaine, tetracaine hydrochloride, tetracaine, chloroprocaine hydrochloride, chloroprocaine, bupivacain hydrochloride, bupivacain, ethyl aminobenzoate or oxethazaine. The specific examples of anti-inflammatory agent, antipruritic agent and wound healing agent include indomethacin, ketoprofen, diclofenac sodium, glycyrrhetinic acid, dimethylisopropylazulene, ichthammol, camphor, crotamiton, lysozyme chloride, d-camphor, dl-camphor, horse chestnut extract, hamamelis extract, Lithospermi Radix extract, mint oil, dl-menthol, 1-menthol, eucalyptus oil, allantoin or aluminum chlorohydroxy-allantoinate. The specific examples of vitamin agent include tocopherol acetate, tocopherol, ergocalciferol, retinol palmitate, retinol acetate, pyridoxine hydrochloride, pyridoxamine hydrochloride, pyridoxal phosphate, riboflavin, riboflavin acetate, vitamin A oil, strong liver oil or liver oil. The specific examples of sulfa drug include sulfadiazine, sulfisomidine or homosulfamine. The specific examples of germicide include isopropylmethylphenol, benzalkonium chloride, chlorhexidine hydrochloride, chlorhexidine gluconate solution, phenol, cetylpyridinium chloride. The specific examples of vasoconstrictor include naphazoline hydrochloride, ephedrine hydrochloride, epinephrine hydrochloride, tetrahydrozoline hydrochloride, phenylephrine hydrochloride or dl-methylephedrine hydrochloride. The specific examples of antihistamic agent include diphenhydramine hydrochloride, diphenhydramine, chlorpheniramine maleate. These drags are commercially available and therefore are easily available. Further, the drag can be used alone or in combination of two or more drugs.

The water-soluble polymer used in the present invention can be any natural polymer, synthetic polymer, biological polymer, inorganic polymer, and homopolymer, copolymer, block polymer or copolymer or the like. The viscosity of the water-soluble polymer is from 0.1 to 1.00 Pa.s, preferably from 1.0 to 7.0 Pa.s, particularly preferably from 1.0 to 5.0 Pa.s under the condition using 20 % by weight aqueous solution of the water-soluble polymer at 37 °C. When a water-soluble polymer having a viscosity of less than 0.1 Pa.s under the condition using 20 % by weight aqueous solution of the water-soluble polymer at 37 °C is used, the viscosity of the polymer is so low that the coated drug-supported particle cannot be adhered to and retained in the mucosa of the affected region or the lower legion of the rectum, and then the coated drug-supported particle is spread to the upper region of the rectum as the time passes. Also, when a water-soluble polymer having a viscosity of more than 10.0 Pa.s is used, the viscosity of the polymer is so high that the production of the coated drug-supported particle becomes difficult.

Any various water-soluble polymers can be used without particular limitation as long as they can be the water-soluble polymers as specified above. The examples of the water-soluble polymer include, for example, nonionic celluloses, vinyl polymer, starches and derivative thereof, natural polysaccharides, natural rubbers, proteins and the like. The specific examples of nonionic celluloses include hydroxypropylcellulose, hydroxymethylpropylcellulose, carboxyethylcellulose, methylcellulose and the like. The specific examples of vinyl polymer include polyvinylpyrrolidone, polyvinyl alcohol, carboxyvinyl polymer and the like. The specific examples of starches and derivative thereof include carboxymethyl starch, starch and the like. The specific examples of natural polysaccharides include xanthan gum, dextran, pullulan and the like. The specific examples of natural rubbers include gum arabic, guar gum, locust bean gum and the like. The specific examples of proteins include gelatin, casein, albumin and the like. Hydroxypropylcelullose is more preferable. These water-soluble polymers are commercially available and therefore are easily available.

The base used in the present invention, in case where the pharmaceutical preparation is in the form of a suppository, is lipophilic or hydrophilic base that is generally used as a suppository base. Lipophilic base includes cacao fat, lanolin fat, hard fat and the like. The specific examples of hard fat include Witepsol, Sapposier, Isocacao, Pharmasol and the like. Hydrophilic base includes polyethylene glycol, glycero gelatin and macrogol. These bases can be used separately or lipophilic base and hydrophilic base can be mixed to use.

In case where the pharmaceutical preparation is in the form of an injection type ointment, a base in general use can be used in the form of a lipophilic ointment. Lipophilic ointment includes oil and fat, fatty acid, higher alcohol and fatty acid triglyceride. The specific examples of oil and fat include olive oil, soybean oil, jojoba oil, rape seed oil, peanut oil, castor oil, mentha oil, coconut oil, cacao oil, palm oil, sesame oil, camellia oil, corn oil, hydrogenated oil, Japan wax, carnauba wax, lanolin oil, bees wax, squalane, squalene, beef tallow, lard, egg yolk oil, spermaceti, fluid paraffin, paraffin, vaselin and the like. The specific examples of fatty acid include oleic acid, palmitic acid and stearic acid. The specific examples of higher alcohol include cetanol and stearyl alcohol. The specific example of fatty acid triglyceride includes Panasate.

Moreover, if necessary, the above-mentioned adrenocortical hormone agent, local anesthetic agent, anti-inflammatory agent, antipruritic agent, wound healing agent, vitamin agent, sulfa drug, germicide, antihistamic agent and the like can be also blended into the base in order to allow the efficacy of a drug to exert immediately after rectal administration of the suppository or injection type ointment. Further, an additive such as an antiseptic and an antioxidant can be also blended into the base. The specific examples of antiseptic include alkyl polyoxybenzoate and sorbitan acid. The specific example of antioxidant includes dibutylhydroxytoluene.

In addition, during making the coated drug-supported particle according to the present invention, an additive to control the releasing property of a drug can be also blended into that particle or onto its surface. The blending of the additive imparts the sustained-release function to the pharmaceutical preparation. The specific examples of additive include amino alkyl methacrylate copolymer RS (trade name: EUDRAGIT RS: manufactured by Rohm Pharma), ethyl cellulose (trade name: ETHOCEL: manufactured by Dow Chem.) and the like. Moreover, if necessary, water dispersion of amino alkyl methacrylate copolymer RS (trade name: EUDRAGIT RS30D: manufactured by Rohm Pharma) or water dispersion of ethyl cellulose (trade name: AQUACOAT: manufactured by Asahi Kasei Corporation, Ltd.) can be also used as a water dispersion type drug-releasing property controlling additive.

Moreover, the above-mentioned coated drug-supported particle wherein said drug is supported by the porous microparticulate carrier and the drug-supported carrier is coated with said water-soluble polymer, has preferably a particle size of from 0.1 to 300 $\mu$m, more preferably from 0.1 to 100 $\mu$m, particularly preferably from 0.1 to 50 $\mu$m. When the coated drug-supported particle is a small particle with a particle size of 300 $\mu$m or less, the particle is dispersed evenly over the mucosa of the affected region in the rectum compared to a larger particle. Furthermore, the particle can adhere to and can be retained at the mucosa due to said water-soluble polymer. Therefore, the particle having the properties above can sustained-release a lot of drugs uniformly throughout the affected region. On the other hand, in case where the particle size of a coated drug-supported particle is more than 300 $\mu$m, when a suppository is produced by dispersing the particles into a base described below, the coating layer of the coated drug-supported particle is damaged during the production of the suppository and the coated drug-supported particle becomes easily sedimented to cause the ununiform distribution of the coated drug-supported particles from the suppository. Consequently, when the suppository is melted by the body temperature or the secretion, the eluting amount of the coated drug-supported particles may be changed and the suppository may be easily cracked. In addition, in case where the coated drug-supported particle is in the form of an injection type ointment, the coated drug-supported particles become difficult to be dispersed into the injection type ointment base uniformly.

Next, a method of producing the pharmaceutical preparation for rectal administration according to the present invention is explained.

Firstly, a drug and a porous microparticulate carrier are blended, for example, in the mass ratio of 1.0 : 0.1 to 10.0, preferably 1.0 : 0.5 to 5.0, and mixed to allow the drug to be supported by the porous microparticulate carrier. In this case, it is preferable that the drug is dissolved in water or organic solvent such as ethanol or a mixed solution of water and organic solvent such as ethanol to mix with the porous microparticulate carrier in order to allow the drug to be adsorbed into pores of the porous microparticulate carrier. Specifically, for example, the drug is dissolved in a solvent in an amount range of from the solvent amount wherein the drug can be adequately dissolved in the solvent to the solvent amount wherein the drug can be adsorbed onto the porous microparticulate carrier, and then the obtained drug solution and the porous microparticulate carrier are mixed. After mixing, the mixture is passed through a sieve of 300 $\mu$m or less and dried for 120 to 600 minutes at the temperature range of the melting point or less of the supported drug or the temperature range that the supported drug is not decomposed.

Then, the resulting drug-supported particle and a water-soluble polymer with a certain viscosity are blended, for example, in the mass ratio of 1.0 : 0.01 to 1.0, preferably 1.0 : 0.05 to 0.5, to allow the drug-supported particle to be coated with the water-soluble polymer. In this case, it is preferable that the water-soluble polymer is dissolved in water or organic solvent such as ethanol or a mixed solution of water and organic solvent such as ethanol to mix with the drug-supported particle in order to allow the surface of the drug-supported particle to be coated with the water-soluble polymer. Specifically, for example, the water-soluble polymer is dissolved in a solvent in an amount range of from the solvent amount wherein the water-soluble polymer can become uniform to the solvent amount wherein the polymer can be adsorbed onto the porous microparticulate carrier, and then the water-soluble polymer solution and the porous microparticulate carrier are mixed. After mixing, the mixture is passed through a sieve of 300 $\mu$m or less and dried for 120 to 600 minutes at the temperature range of the melting point or less of the supported drug or the temperature range that the supported drug is not decomposed.

Then, the resulting coated drug-supported particle is blended into a base, for example, in the amount of 1 to 30 % by weight, preferably 5 to 20 % by weight based on the base, and mixed to form a suppository, or in the case of an injection type ointment, to fill the obtained mixture in the semi-solid form into a injection container. During blending, two or more kinds of coated drug-supported particles wherein the drugs are different each other can be blended into the base. Specifically, in the case of a suppository, the suppository can be produced according to the dissolution method and cold pressing method in Suppository Preparation Method described in Japanese Pharmacopoeia. Specifically, the suppository can be produced by the melting method wherein desired amount of a drug which may be same as or different from the drug of the coated drug-supported particle and a generally-used additive, if necessary, can be also added, the resulting blend is mixed, poured into a mold and cooled to solidify, or by the cold pressing method wherein the coated drug-supported particle and the base are mixed in the mass ratio as stated above and powdered uniformly, and then formed the suppository by using the suppository press machine. In the case of an injection type ointment, the injection type ointment can be produced according to Ointment Preparation Method described in Japanese Pharmacopoeia. Specifically, the ointment can be produced by mixing desired amount of a drug which may be same as or different from the drug of the coated drug-supported particle and a generally-used additive, if necessary, with the coated drug-supported particle, adding the remaining base, and stirring until all substances are homogenized.

The present invention is illustrated in further detail by the following Reference Examples and Examples, but the present invention is not limited to these examples.

[Reference examples]

[Viscosity test and adhesion test of water-soluble polymer]

The viscosity test and adhesion test were carried out for the following water-soluble polymers:

Hydroxypropylcellulose (H.P.C-M, H.P.C-L and H.P.C-SSL: manufactured by Nippon Soda);
Methylcellulose (M.C SM-15: manufactured by Shin-Etsu Chemical);
Polyvinylpyrrolidone (P.V.P-K90 and P.V.P-K90: manufactured by BASF).
Sodium alginate (Sodium alginate I-8: manufactured by Kimitsu Chemicals);
Polyethylene glycol (P.E.G 6000: manufactured by NOF Corporation).
Carboxyvinyl polymer (Carbopole 934P: manufactured by BFGoodrich).

1. Viscosity Test Method

Each of the above-mentioned water-soluble polymers was dissolved in water to prepare 20 % by weight aqueous solution and its viscosity was measured. The viscosity (Pa.s) of the 20 % by weight aqueous solution of the water-soluble polymer at 37 °C was measured using E-type viscometer (manufactured by Tokyo Keiki), and the result is shown in Table 1 below.

2. Adhesion Test Method

Cellulose film (VISKING SEAMLEEE CELLULOSE TUBE:
manufactured by HANDEX) which was cut into the size of 5 x 5 cm and was moistened with a purified water at 37 °C, was coated with a certain weight $W_1$ (0.2g) of a water-soluble polymer, and left to stand for 5 minutes. After standing, the cellulose film was washed by shaking it up and down vigorously for 10 times in a purified water (37 °C). After washing, the cellulose film was dried under vacuum (at 70 °C, 76 cmHg, 24h) and the remaining weight $W_2$ of the water-soluble polymer on the cellulose film was obtained to calculate the adhesion rate (%) by the following equation.
The result is shown in Table 1 below.

```
Adhesion rate (%)

= (W₂ - weight of dry cellulose film) / W₁ x 100
```

Table 1. Viscosity and Adhesion rate of Water-soluble polymer

|  | Water-soluble polymer | Viscosity (Pa.s) | Adhesion rate (%) |
|---|---|---|---|
| Ref. Ex.1 | H.P.C-M | 5.012 | 88.9 |
| Ref. Ex.2 | M.C.SM15 | 6.320 | 62.0 |
| Ref. Ex.3 | Carbopole 934P | 5.560 | 68.5 |
| Ref. Ex.4 | Sodium alginate I-8 | 5.008 | 86.1 |
| Ref. Ex.5 | H.P.C-L | 3.430 | 88.2 |
| Ref. Ex.6 | P.V.P-K90 | 1.740 | 60.1 |
| Ref. Ex.7 | H.P.C-SSL | 0.142 | 43.5 |
| Ref. Ex.8 | PEG6000 | 0.015 | 14.8 |
| Ref. Ex.9 | P.V.P-K25 | 0.011 | 37.2 |

Reference Examples 1 to 7 had high viscosity and their adhesion rates were high depending on their viscosity. When water-soluble polymers with high viscosity of Reference Examples 1 to 7 are applied to the rectal mucosa, they are adhered to the rectal mucosa well so that they are suitable for the coating agents of the coated drug-supported particles used in the present invention.

[Examples]

The suppositories and the injection type ointments of Examples 1 to 8 were produced by the following method.
Here, the hydroxypropylcellulose (H.P.C-L :
manufactured by Nippon Soda) used as a water-soluble polymer in Examples 1 to 8 is the hydroxypropylcellulose of foregoing Reference Example 5 (the viscosity under the condition using 20 % by weight aqueous solution of the hydroxypropylcellulose at 37 °C: 3.430 Pa.s).

[Example 1]

Sixty mg of lidocaine hydrochloride which was a drug to be supported on a porous microparticulate carrier was dissolved in 120 ml of 70 % ethanol, mixed well with 80 mg of light silicic acid anhydride which was a porous microparticulate carrier (Adsolider 101: Freund Corporation, 3.5 $\mu$m of the particle size, 300 m$^2$/g of the specific surface area, 3.4 mL/g of the absorbability of oil), passed through a Japanese Pharmacopoeia standard No. 50 sieve, and dried by blowing air at 70 °C for 360 minutes. The resulting drug-supported particles (140 mg) and an ethanol solution of a water-soluble polymer wherein 20 mg of hydroxypropylcellulose (H.P.C-L : Nippon Soda) had been dissolved in 120 mg of 95 % ethanol were mixed well, passed through the No. 50 sieve to be of 10 $\mu$m of the mean particle size, and then dried by blowing air at 70 °C for 360 minutes to yield coated drug-supported particles. 1519 mg of Witepsol W-35, i.e. a base, was heated (at 50 to 60 °C) to dissolve and then 10 mg of diphenhydramine hydrochloride, 1 mg of prednisolone acetate, 10 mg of allantoin and 50 mg of tocopherol acetate which were drugs or the like to be mixed into base were uniformly dispersed in the base with stirring and cooled to about 40 °C. Then, 160 mg of the coated drug-supported particles were uniformly dispersed into the base, and then filled into a suppository container to obtain the suppository (spindle-shaped, 1750 mg of the weight) of the present invention.

[Example 2]

Sixty mg of lidocaine hydrochloride which was a drug to be supported on a porous microparticulate carrier was dissolved in 120 ml of 70 % ethanol, mixed well with 80 mg of magnesium aluminometasilicate which was a porous microparticulate carrier (Neusilin UFL$_2$: Fuji Chemical Industry, 1.6 $\mu$m of the particle size, 260 m$^2$/g of the specific surface area, 3.2 mL/g of the absorbability of oil), passed through the No. 50 sieve, and dried by blowing air at 70 °C for 360 minutes. The resulting drug-supported particles (140 mg) and an ethanol solution of a water-soluble polymer wherein 20 mg of hydroxypropylcellulose (H.P.C-L: Nippon Soda) had been dissolved in 120 mg of 95 % ethanol were mixed well, passed through the No. 50 sieve to be of 10 $\mu$m of the mean particle size, and then dried by blowing air at 70 °C for 360 minutes to yield coated drug-supported particles. Further, the similar procedures as those in Example 1 were repeated to obtain the suppository of the present invention.

[Example 3]

Sixty mg of lidocaine hydrochloride which was a drug to be supported on a porous microparticulate carrier was dissolved in 120 ml of 70 % ethanol, mixed well with the mixture of 50 mg of light silicic acid anhydride (Adsolider 101: Freund Corporation, 3.5 $\mu$m of the particle size, 300 m$^2$/g of the specific surface area, 3.4 mL/g of the absorbability of oil) and 30 mg of magnesium aluminometasilicate (Neusilin UFL$_2$ : Fuji Chemical Industry) which were porous microparticulate carriers, passed through the No. 50 sieve, and dried by blowing air at 70 °C for 360 minutes. The resulting drug-supported particles (140 mg) and an ethanol solution of a water-soluble polymer wherein 20 mg of hydroxypropylcellulose (H.P.C-L: Nippon Soda) had been dissolved in 120 mg of 95 % ethanol were mixed well, passed through the No. 50 sieve to be of 10 $\mu$m of the mean particle size, and then dried by blowing air at 70 °C for 360 minutes to yield coated drug-supported particles. Further, the similar procedures as those in Example 1 were repeated to obtain the suppository of the present invention.

[Example 4]

Sixty mg of lidocaine hydrochloride and 10 mg of diphenhydramine hydrochloride which were drugs to be supported on a porous microparticulate carrier were dissolved in 120 ml of 70 % ethanol, mixed well with 80 mg of light silicic acid anhydride which was a porous microparticulate carrier (Adsolider 101: Freund Corporation, 3.5 $\mu$m of the particle size, 300 m$^2$/g of the specific surface area, 3.4 mL/g of the absorbability of oil), passed through the No. 50 sieve, and dried by blowing air at 70 °C for 360 minutes. 150 mg of the drug-supported particle which was supporting the lidocaine hydrochloride and the diphenhydramine hydrochloridean on it and an ethanol solution of a water-soluble polymer wherein 20 mg of hydroxypropylcellulose (H.P.C-L: Nippon Soda) was dissolved in 120 mg of 95 % ethanol were mixed well, passed through the No. 50 sieve to be of 10 $\mu$m of the mean particle size, and then dried by blowing air at 70 °C for 360 minutes to yield coated drug-supported particles. 1519 mg of Witepsol W-35, i.e. a base, was heated (at 50 to 60 °C) to dissolve and then 1 mg of prednisolone acetate, 10 mg of allantoin and 50 mg of tocopherol acetate which were drugs or the like to be mixed into base were uniformly dispersed in the base with stirring and cooled to about 40 °C. Then, 170 mg of the coated drug-supported particles were uniformly dispersed into the base, and then filled into a suppository container to obtain the suppository (spindle-shaped, 1750 mg of the weight) of the present invention.

[Example 5]

Sixty mg of lidocaine hydrochloride and 10 mg of diphenhydramine hydrochloride, which were drugs to be supported on a porous microparticulate carrier, each was dissolved in 120 ml of 70 % ethanol, separately mixed well with each 80 mg of light silicic acid anhydride which was a porous microparticulate carrier (Adsolider 101: Freund Corporation, 3.5 $\mu$m of the particle size, 300 m$^2$/g of the specific surface area, 3.4 mL/g of the absorbability of oil), passed through the No. 50 sieve, and dried by blowing air at 70 °C for 360 minutes. Each of the resulting lidocaine hydrochloride-supported particles (140 mg) and diphenhydramine hydrochloride-supported particles (90 mg) and each ethanol solution of a water-soluble polymer wherein 20 mg of hydroxypropylcellulose (H.P.C-L: Nippon Soda) had been dissolved in 120 mg of 95 % ethanol were separately mixed well, passed through the No. 50 sieve to be of 10 $\mu$m of the mean particle size of the lidocaine hydrochloride-supported particles and 10 $\mu$m of the mean particle size of the diphenhydramine hydrochloride-supported particles, and then dried by blowing air at 70 °C for 360 minutes to yield coated drug-supported particles. 1419 mg of Witepsol W-35, i.e. a base, was heated (at 50 to 60 °C) to dissolve and then 1 mg of prednisolone acetate, 10 mg of allantoin and 50 mg of tocopherol acetate which were drugs or the like to be mixed into base were uniformly dispersed in the base with stirring and cooled to about 40 °C. Then, 160 mg of the lidocaine hydrochloride-supported particles and 110mg of diphenhydramine hydrochloride-supported particles were uniformly dispersed into the base, and then filled into a suppository container to obtain the suppository (spindle-shaped, 1750 mg of the weight) of the present invention.

[Example 6]

Sixty mg of lidocaine, which was a drug to be supported on a porous microparticulate carrier, was dissolved in 120 ml of 95 % ethanol, mixed well with 80 mg of light silicic acid anhydride which was a porous microparticulate carrier (Adsolider 101: Freund Corporation, 3.5 $\mu$m of the particle size, 300 m$^2$/g of the specific surface area, 3.4 mL/g of the absorbability of oil), passed through the No. 50 sieve, and dried by blowing air at 60 °C for 360 minutes. The resulting drug-supported particles (140 mg) and a water-soluble polymer solution wherein 20 mg of hydroxypropylcellulose (H.P.C-L: Nippon Soda) had been dissolved in 120 mg of purified water were mixed well, passed through the No. 50 sieve to be of 10 $\mu$m of the mean particle size, and then dried by blowing air at 60 °C for 360 minutes to yield coated drug-supported particles. Further, the similar procedures as those in Example 1 were repeated to obtain the suppository of the present invention.

[Example 7]

Sixty mg of lidocaine, which was a drug to be supported on a porous microparticulate carrier, was dissolved in 120 ml of 95 % ethanol, mixed well with 80 mg of magnesium aluminometasilicate which was a porous microparticulate carrier (Neusilin UFL$_2$: Fuji Chemical Industry, 1.6 μm of the particle size, 260 m$^2$/g of the specific surface area, 3.2 mL/g of the absorbability of oil), passed through the No. 50 sieve, and dried by blowing air at 60 °C for 360 minutes. The resulting drug-supported particles (140 mg) and a water-soluble polymer solution wherein 20 mg of hydroxypropylcellulose (H.P.C-L: Nippon Soda) had been dissolved in 120 mg of purified water were mixed well, passed through the No. 50 sieve to be of 10 μm of the mean particle size, and then dried by blowing air at 60 °C for 360 minutes to yield coated drug-supported particles. Further, the similar procedures as those in Example 1 were repeated to obtain the suppository of the present invention.

[Example 8]

Sixty mg of lidocaine hydrochloride which was a drug to be supported on a porous microparticulate carrier was dissolved in 120 ml of 70 % ethanol, mixed well with 80 mg of light silicic acid anhydride which was a porous microparticulate carrier (Adsolider 101: Freund Corporation, 3.5 μm of the particle size, 300 m$^2$/g of the specific surface area, 3.4 mL/g of the absorbability of oil), passed through a Japanese Pharmacopoeia standard No. 50 sieve, and dried by blowing air at 70 °C for 360 minutes. The resulting drug-supported particles (140 mg) and an ethanol solution of a water-soluble polymer wherein 20 mg of hydroxypropylcellulose (H.P.C-L: Nippon Soda) had been dissolved in 120 mg of 95 % ethanol were mixed well, passed through the No. 50 sieve to be of 10 μm of the mean particle size, and then dried by blowing air at 70 °C for 360 minutes to yield coated drug-supported particles. 1832 mg of white vaseline, i.e. a base, was heated (at 50 to 60 °C) to dissolve and then 2 mg of dl-methylephedrine hydrochloride, 1 mg of prednisolone and 5 mg of aluminum chlorohydroxy-allantoinate which were drugs or the like to be mixed into base were uniformly dispersed in the base with stirring and cooled to about 40 °C. Then, 160 mg of the coated drug-supported particles were uniformly dispersed into the base, and then filled into a container for an injection type ointment to obtain the ointment (2 g) of the present invention.

[Comparative Example 1]

The suppository was produced in the same manner as Example 1 except for using 20 mg of polyethylene glycol (P.E.G 6000: NOF Corporation) in place of 20 mg of hydroxypropylcellulose (H.P.C-L: Nippon Soda) as a water-soluble polymer. Here, the polyethylene glycol (P.E.G 6000: NOF Corporation) used as a water-soluble polymer in the comparative example corresponded to the polyethylene glycol in the reference example 8 (the viscosity under the condition using 20 % by weight aqueous solution of polyethylene glycol at 37 °C: 0.015 Pa.s).

[Test Example 1]

[Cellulose Tube Adhesion Test]

A cellulose tube was used to create a pseudo rectal environment, and the adhesion and retainability of the suppositories of Example 1 and Comparative example 1 under such environment were evaluated.

[Test Method]

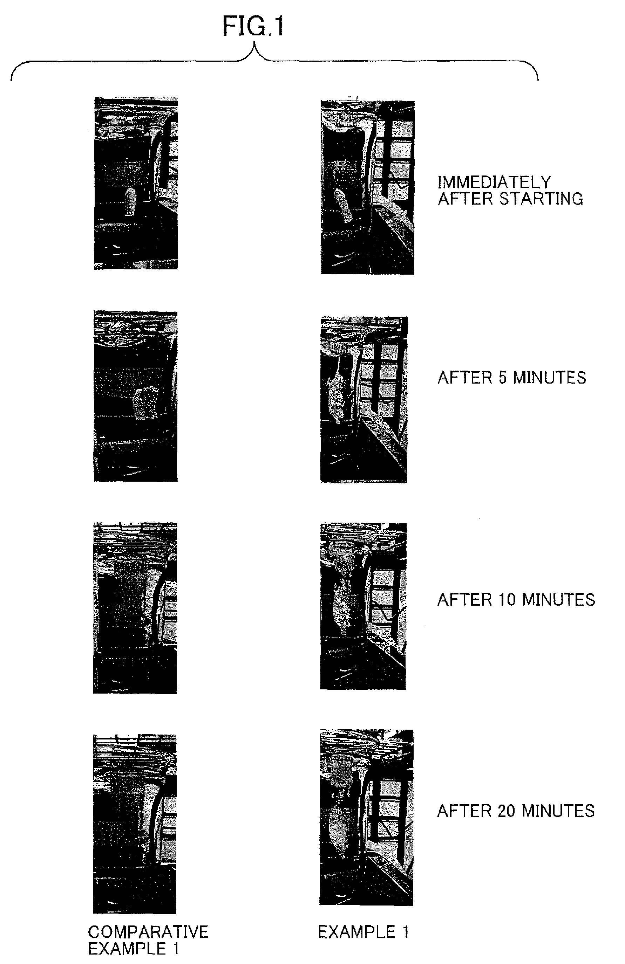

Size φ 14 x 25 cm of cellulose tube (VISKING SEAMLEEE CELLULOSE TUBE : manufactured by HANDEX) was sealed at the bottom, the suppository of Example 1 or the suppository of Comparative example 1 and 5ml of purified water were placed into the tube, and its upper end was sealed. This cellulose tube was placed into a beaker filled with water maintained at 37 °C and the adhesion of coated drug-supported particles to the inner wall of the cellulose tube was observed after a given period of time. The observations of the adhesion were carried out immediately after the test starting, after 5 minutes, after 10 minutes and after 20 minutes. Here, the coated drug-supported particles were colored blue such that they can be easily observed. The result is shown in Figure 1.

For the suppository of Example 1, the coated drug-supported particles were released from the suppository and adhered to the inner wall of the cellulose tube after 10 minutes from the test starting or later (the right-hand side in Figure 1).

On the other hand, for the suppository of Comparative example 1, the coated drug-supported particles were not adhered to the inner wall of the cellulose tube, and then they were transferred to the upper region in the cellulose tube after 10 minutes from the test starting or later (the left-hand side in Figure 1).

These results show that the pharmaceutical preparation for rectal administration of the present invention allows the coated drug-supported particles to be adhered to and retained in the affected region.

[Test Example 2]

[Determination of Amount of Drug in Rectal Tissue]

The suppository of Example 1 or the suppository of Comparative example 1 was administered to a rat and the concentration of drugs (lidocaine hydrochloride) in the rat rectal tissue after predetermined time was evaluated.

[Test Method]

In this test, each suppository produced as described in Example 1 and Comparative example 1 was mold into each one with the size capable of being administered through rat's anus (cylinder-shaped, 100 mg of the weight, the concentration of lidocaine hydrochloride was 3.4 mg per suppository) to use.

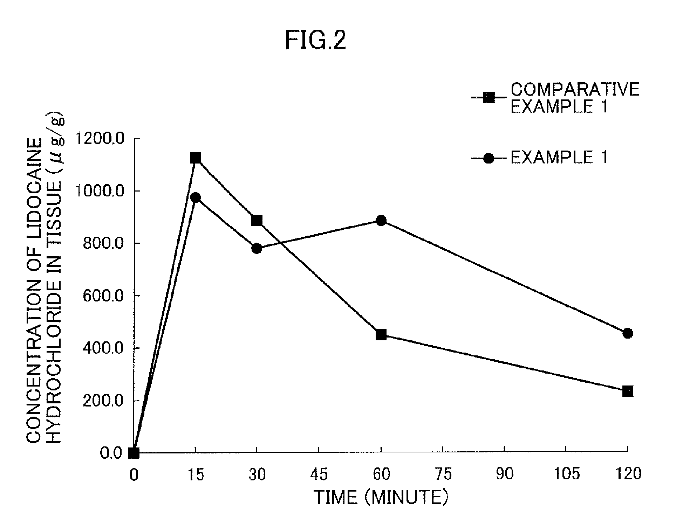

Each of above suppositories was administered to each rat fasted for 48 hours, and the anus was immediately sealed by an instant adhesive (Aron Alpha®) to prevent the suppository from leaking. After a certain period of time, the rectum was excised, washed sufficiently by physiological saline solution, and then the rectal tissue of a section of 4 cm from the anal region was collected. The collected rectal tissue was homogenated using physiological saline solution having the amount of 100 times the weight of the collected tissue, and then the lidocaine hydrochloride in the tissue was extracted. The test solution was prepared from the extract and the determination of the amount of the lidocaine hydrochloride in 1 g of the rectal tissue was carried out using a high-performance liquid chromatography (HPLC method). Here, the excision of the rectum was performed 15 minute, 30 minute, 60 minutes and 120 minutes after the administration of the suppository. The result is shown in Figure 2.

Sixty minutes after the administration or later, the suppository of Example 1 exhibited higher concentration of lidocaine hydrochloride in the rectal tissue than that of the suppository of Comparative example 1. The result of Example 1 shows that the suppository of Example 1 allowed the drug-supported particles to adhere to and retain at the intestinal mucosa so that the concentration of drugs in the rectal tissue could maintain for a long time. On the other hand, the result of Comparative example 1 shows that the suppository of Comparative Example 1 did not allow the drug-supported particles to adhere to and retain at the intestinal mucosa so that the drug-supported particles would move following the movement of the suppository, and consequently the concentration of drugs in the rectal tissue became low.

INDUSTRIAL APPLICABILITY

By appropriately selecting the drug to be supported on the porous microparticulate carrier, the base and the like, a suppository and an injection type ointment to be applied to the vagina, urethra, nasal cavity or the like as well as the rectum (anus) can be prepared.

**Claims**

1. A pharmaceutical preparation for rectal administration comprising a coated drug-supported particle dispersed in a base, **characterized in that** said coated drug-supported particle is what a drug-supported porous microparticulate carrier is coated with a water-soluble polymer having a viscosity of from 0.1 to 10.0 Pa.s under the condition using 20 % by weight aqueous solution of the water-soluble polymer at 37 °C.

2. The pharmaceutical preparation for rectal administration according to claim 1, wherein said water-soluble polymer has a viscosity of from 1.0 to 7.0 Pa.s.

3. The pharmaceutical preparation for rectal administration according to claim 1 or 2, wherein said water-soluble polymer has a viscosity of from 1.5 to 5.0 Pa.s.

4. The pharmaceutical preparation for rectal administration according to any one of claims 1 to 3, wherein said coated drug-supported particle has a particle size of from 0.1 to 300 $\mu$m.

5. The pharmaceutical preparation for rectal administration according to any one of claims 1 to 4, wherein said coated drug-supported particle has a particle size of from 0.1 to 100 $\mu$m.

6.  The pharmaceutical preparation for rectal administration according to any one of claims 1 to 5, wherein said coated drug-supported particle has a particle size of from 0.1 to 50 $\mu$m.

7.  The pharmaceutical preparation for rectal administration according to any one of claims 1 to 6, wherein said water-soluble polymer is hydroxypropylcellulose.

8.  The pharmaceutical preparation for rectal administration according to any one of claims 1 to 7, wherein said pharmaceutical preparation for rectal administration is a suppository.

9.  The pharmaceutical preparation for rectal administration according to any one of claims 1 to 7, wherein said pharmaceutical preparation for rectal administration is an injection type ointment.

# FIG.1

IMMEDIATELY AFTER STARTING

AFTER 5 MINUTES

AFTER 10 MINUTES

AFTER 20 MINUTES

COMPARATIVE EXAMPLE 1

EXAMPLE 1

FIG.2

**INTERNATIONAL SEARCH REPORT**

| | International application No. |
|---|---|
| | PCT/JP2004/012870 |

| A. CLASSIFICATION OF SUBJECT MATTER |
|---|
| Int.Cl⁷ A61K9/02, A61K9/06, A61K47/38 |

According to International Patent Classification (IPC) or to both national classification and IPC

| B. FIELDS SEARCHED |
|---|
| Minimum documentation searched (classification system followed by classification symbols) |
| Int.Cl⁷ A61K9/02, A61K9/06, A61K47/38 |

Documentation searched other than minimum documentation to the extent that such documents are included in the fields searched

Electronic data base consulted during the international search (name of data base and, where practicable, search terms used)
CAP(STN), WPIDS(STN)

| C. DOCUMENTS CONSIDERED TO BE RELEVANT | | |
|---|---|---|
| Category* | Citation of document, with indication, where appropriate, of the relevant passages | Relevant to claim No. |
| X<br>Y | JP 1-143825 A (TAISHO PHARM CO., LTD.),<br>06 June, 1989 (06.06.89),<br>Full text; Claims; page 1, lower right column,<br>middle part, examples 1, 2<br>(Family: none) | 1-6,8,9<br>1-9 |
| X<br>Y | WO 02/24161 A1 (TAISHO PHARM CO., LTD.),<br>28 March, 2002 (28.03.02),<br>Full text; Claims; page 3, lines 7 to 28;<br>examples 1, 2, 5; comparative examples 1 to 3<br>& AU 2001/88039 B &  EP 1319397 A1<br>& US 2003/185861 A | 1-6,8,9<br>1-9 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>30 November, 2004 (30.11.04) | Date of mailing of the international search report<br>28 December, 2004 (28.12.04) |
|---|---|
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

| International application No. |
|---|
| PCT/JP2004/012870 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | WO 99/17737 A1 (TAISHO PHARM CO., LTD.),<br>15 April, 1999 (15.04.99),<br>Full text; Claims; page 3, lines 7 to 17;<br>examples 8 to 10; comparative examples 7 to 9;<br>test example 3<br>& ZU 9894576 B        & EP 1022019 A1<br>& US 6210698 A | 1-6,8,9<br>1-9 |
| X<br>Y | JP 9-132521 A (TAISHO PHARM CO., LTD.),<br>20 May, 1997 (20.05.97),<br>Full text; Claims; Par. Nos. [0002], [0003];<br>examples 1, 2<br>& WO 97/9035 A1        & AU 9668891 B<br>& EP 848948 A1        & US 6136337 A | 1-6,8,9<br>1-9 |
| X<br>Y | WO 00/29026 A1 (LEXICON GENETICS INC.),<br>25 May, 2000 (25.05.00),<br>Full text; page 3, lines 6 to 16; comparative<br>example 1; test example 1<br>& US 2002/102683 A        & AU 2002/11347 B<br>& EP 1328623 A2        & JP 2004-524818 A | 1-6,8,9<br>1-9 |
| X | JP 7-304669 A (TAISHO PHARM CO., LTD.),<br>21 November, 1995 (21.11.95),<br>Full text; Claims; examples 1, 2<br>(Family: none) | 1-6,8,9 |
| X | JP 59-55817 A (CILAG AG INTERNATIONAL),<br>31 March, 1984 (31.03.84),<br>Full text; page 2, upper left column, middle<br>part to upper right column; example 2<br>& GB 2126086 A        & EP 103995 A1<br>& AU 8318334 B        & CA 1207231 A1 | 1-6,8,9 |
| Y | JP 4-91020 A (Nippon Shinyaku Co., Ltd.),<br>24 March, 1992 (24.03.92),<br>Full text; Claims; page 1, lower right column,<br>lines 2 to 12; page 2, lower left column, 2nd<br>line from the bottom to page 3, upper left<br>column, line 1; examples<br>(Family: none) | 1-9 |
| Y | JP 63-280016 A (TAISHO PHARM CO., LTD.),<br>17 November, 1988 (17.11.88),<br>Full text; page 1, lower right column, middle<br>part; examples 1, 2<br>(Family: none) | 1-9 |
| Y | JP 2003-119119 A (Pola Chemical Industries<br>Inc.),<br>23 April, 2003 (23.04.03),<br>Full text; Claims; examples 1 to 8;<br>particularly, example 4<br>(Family: none) | 1-9 |

Form PCT/ISA/210 (continuation of second sheet) (January 2004)

**INTERNATIONAL SEARCH REPORT**

| International application No. |
| --- |
| PCT/JP2004/012870 |

**C (Continuation).  DOCUMENTS CONSIDERED TO BE RELEVANT**

| Category* | Citation of document, with indication, where appropriate, of the relevant passages | Relevant to claim No. |
| --- | --- | --- |
| Y | JP 6-40889 A  (NOF Corp.),<br>15 February, 1994 (15.02.94),<br>Full text; Claims; Par. Nos. [0003], [0004];<br>examples<br>(Family: none) | 1-9 |
| Y | JP 7-179345 A  (Yoshitomi Pharmaceutical<br>Industries, Ltd.),<br>18 July, 1995 (18.07.95),<br>Full text; Par. No. [0030]; referential<br>example 1<br>(Family: none) | 1-9 |
| A | JP 2001-261551 A  (Amato Pharmaceutical Products<br>Ltd.),<br>26 September, 2001 (26.09.01),<br>Full text; Claims<br>(Family: none) | 1-9 |
| A | JP 2002-537316 A  (DONG WHA PHARM. IND. CO.,<br>LTD.),<br>05 November, 2002 (05.11.02),<br>Full text; Claims; Par. No. [0015]; examples<br>& WO 00/50005 A1      & EP 1158957 A1<br>& US 6488954 A | 1-9 |

Form PCT/ISA/210 (continuation of second sheet) (January 2004)